# EUROPÄISCHE PATENTSCHRIFT

(11) **EP 0 888 545 B1**
(45) Veröffentlichungstag und Bekanntmachung des Hinweises auf die Patenterteilung: **23.01.2002**
(21) Anmeldenummer: 97920534.1
(22) Anmeldetag: 13.03.1997
(51) Int. Cl.: G01N 33/543, A61K 47/02, A61K 49/00

(54) **SUPERPARAMAGNETISCHE TEILCHEN MIT VERGRÖSSERTER R 1-RELAXIVITÄT, VERFAHREN ZUR HERSTELLUNG UND DEREN VERWENDUNG**
SUPER-PARAMAGNETIC PARTICLES WITH INCREASED R 1 RELAXIVITY, PROCESSES FOR PRODUCING SAID PARTICLES AND USE THEREOF
PARTICULES SUPERPARAMAGNETIQUES A RELAXIVITE R 1 ACCRUE, LEUR PROCEDE DE FABRICATION ET LEUR UTILISATION

(30) Priorität: 18.03.1996 DE 19612001
(43) Veröffentlichungstag der Anmeldung: 07.01.1999
(73) Patentinhaber: Pilgrimm, Herbert Dr., 14169 Berlin (DE)
(72) Erfinder: Pilgrimm, Herbert Dr., 14169 Berlin (DE)
(74) Vertreter: Walter, Wolf-Jürgen
(86) Internationale Anmeldenummer: DE9700578
(87) Internationale Veröffentlichungsnummer: WO9735200

(56) Entgegenhaltungen:
- EP-A- 0 284 549
- EP-A- 0 580 878
- WO-A-89/11154
- WO-A-90/01899
- WO-A-90/07380
- DE-A- 4 309 333
- DE-A- 4 427 821
- MAGNETIC RESONANCE IN MEDICINE, Bd. 24, Nr. 1, 1992, Seiten 75-84, XP002035968 D. POULIQUEN ET AL.: "Investigation of the magnetic properties of Iron Oxide nanoparticles used as contrast agent for MRI."

## Beschreibung

Die Erfindung betrifft superparamagnetische Teilchen mit vergrößerter R1-Relaxivität, die als Diagnostika in der NMR-Tomographie sowie als pharmakologisch wirksame Substanz eingesetzt werden können. Die Erfindung betrifft auch ein Herstellungsverfahren und die Verwendung der neuen Teilchen.

Aus der WO90/01899 sind superparamagnetische Eisenoxidaggregate für die NMR-Tomografie bekannt mit Teilchengrößen von 10-4000 nm und stabilisiert mit makromolekularen Substanzen.

Im Patent EP-B-0284549 werden superparamagnetische Eindomänenteilchen aus Eisenoxid, Eisenmischoxid oder Eisen mit einer Teilchengröße im Bereich zwischen 3 und 20 Nanometer beschrieben, die auf ihrer Oberfläche organische Substanzen der Gruppe der phosphat-, diphosphat-, polyphosphat-, thiophosphat-, phosphonat- oder thiophosphonatgruppenhaltigen Polyalkylenglykole, phosphatgruppenhaltigen Nucleotide, deren oligomere oder deren Polymere sowie phosphatgruppenhaltigen Kohlenhydrate chemisch gebunden tragen, die weitere Bindungsstellen haben können.

In der DE-A-4309333 werden stabile, abbaubare Aggregate mit einer Teilchengröße im Bereich zwischen 10 und 1000 Nanometer mit definiertem Verhalten im Magnetfeld beschrieben, wobei die Aggregate aus mehreren kleinen superparamagnetischen Eindomänen-teilchen aus Eisenoxid, Eisenmischoxid oder Eisen mit einer Teilchengröße im Bereich zwischen 3 und 20 Nanometer bestehen, die auf ihrer Oberfläche Substanzen der Gruppe der phosphat-, diphosphat-, polyphosphat-, thiophosphat-, phosphonat- oder thiophosphonatgruppenhaltigen Polyalkylenglykole, Kohlenhydrate oder der phosphatgruppenhaltigen Nucleotide, deren Oligomere oder deren Polymere chemisch gebunden tragen.

In WO 96/03653 werden superparamagnetische Teilchen, die aus superparamagnetischen Eindomänenteilchen und Aggregaten von superparamagnetischen Eindomänenteilchen bestehen, die auf ihrer Oberfläche organische Substanzen gebunden haben. Die superpara-magnetischen Teilchen setzen sich aus einem Gemisch von kleinen superparamagnetischen Eindomänenteilchen mit einer Teilchengröße im Bereich zwischen 3 und 50 Nanometer und stabilen, abbaubaren Aggregaten aus kleinen superparamagnetischen Eindomänenteilchen mit einer Teilchengröße im Bereich zwischen 10 und 1000 Manometer zusammen und bestehen aus Eisenhydroxid, Eisenoxidhydrat,Eisenoxid, Eisenmischoxid oder Eisen, die auf ihrer Oberfläche mono- und/oder polyhydroxylgruppenhaltige aromatische Substanzen, Polyglycerine, aminosäurenhaltige Substanzen, silikatgruppenhaltige Substanzen der Orthokieselsäure und deren Kondensationsprodukte und phosphatgruppenhaltige Substanzen der Ortho- oder Metaphosphorsäure und deren Kondensationsprodukte gebunden tragen.

Nach dem Stand der Technik können superparamagnetische Eindomänenteilchen mit einer Teilchengröße im Bereich zwischen 3 und 50 Nanometer hergestellt und z.B. als Kontrastmittel für die NMR-Tomographie mit bevorzugten Teilchendurchmessern im Bereich von 5-20 nm eingesetzt werden. Diese Teilchen führen in den Körperregionen, in denen sie sich angereichert haben, aufgrund einer stärkeren Verkürzung der T2-Relaxationszeit, zur Signalverdunklung. Man spricht deshalb auch bei superparamagnetischen Eindomänenteilchen von "negativen" NMR-Kontrastmitteln. Da die T-Relaxationszeit umgekehrt proportional der Relaxivität R ist, vergrößert sich die Relaxivität R mit Verkürzung der T-Relaxationszeit. Die T2-Relaxationszeit hängt linear vom Teilchendurchmesser der Magnetteilchen ab. Je größer der Teilchendurchmesser, desto stärker verkürzt sich die T2-Relaxationszeit und um so geringer ist die benötigte Teilchenkonzentration, um einen "negativen" Kontrast zu erreichen. Der Kontrastmittelbedarf liegt im Bereich von 10 bis 20 mmol Fe/kg Körpermasse, und somit gegenüber dem bisherigen "positiven" NMR-Kontrastmittelbedarf, auf der Basis von Gadolinium-Chelaten, wesentlich niedriger.

Die T1-Relaxationszeit solcher "negativer" Kontrastmittel verkürzt sich nicht wesentlich, so daß auch keine bemerkenswerte Signalverstärkung in der NMR-Tomographie in den Körperregionen, in denen sie sich angereichert haben, erfolgt. Die Signalaufhellung im Gewebe ist gering. Für viele Anwendungszwecke wäre es günstig, superparamagnetischen Eindomänenteilchen auch mit verstärkter "positiver" NMR-Kontrastmittel-wirkung einsetzen zu können.

Will man T1-gewichtete NMR-Tomographie betreiben, muß das Verhältnis der Relaxivitäten R2/R1 so klein wie möglich, mindestens kleiner 5 sein.

Mit Verringerung des Teilchendurchmessers läßt sich die R1-Relaxivität nicht wesentlich verändern, dafür jedoch die R2-Relaxivität verkleinern. Bei sehr kleinen superparamagnetischen Eindomänenteilchen verringert sich das Verhältnis der Relaxivitäten R2/R1 so stark, daß man T1-gewichtete NMR-Tomographie betreiben kann. Da superparamagnetische Eindomänenteilchen schon in sehr geringen Konzentrationen die Signalintensität beeinflussen, können sie den bisherigen paramagnetischen Kontrastmitteln überlegen sein.

Der Erfindung liegt die Aufgabe zugrunde, sehr kleine superparamagnetische Eindomänenteilchen zu entwickeln, die z.B. als Kontrastmittel für die T1-gewichtete NMR-Tomographie einsetzbar sind.

Erfindungsgemäß kann das durch superparamagnetische Eindomänenteilchen mit vergrößerter R1-Relaxivität und mit Oberflächen-Stabilisatorsubstanzen erreicht werden, dadurch gekennzeichnet, daß Teilchen aus Eisenhydroxid, Eisenoxidhydrat, Eisenoxid, Eisenmischoxid oder Eisen eine Teilchengröße im Bereich zwischen 1 und 10 Nanometer haben, mit einem mittleren Teilchendurchmesser d₅₀ von 2 bis 4 Nanometer; eine vergrößerte R1-Relaxivität im Bereich von 2 bis 50 haben; ein Verhältnis der Relaxivitäten R2/R1 kleiner 5 haben; und auf ihrer Oberfläche Stabilisatorsubstanzen tragen, die eine Aggregation und Sedimentation im Schwerefeld oder in einem Magnetfeld verhindern; und gegebenenfalls einen Gehalt an gewebespezifischer Bindungssubstanz, pharmakologisch wirksamer Substanz, pharmakologisch wirksamen Zellen, pharmakologisch wirksamen Komplexbildner, zellfusionvermittelnder Substanz oder gentransfervermittelnder Substanz; sowie Gemischen davon aufweisen.

Es wurden verschiedene Möglichkeiten gefunden, dieses Ziel zu erreichen:
1. durch eine Verringerung der magnetischen Suszeptibilität der superparamagnetischen Eindomänenteilchen,
2. durch eine Verringerung des Teilchendurchmessers der superparamagnetischen Eindomänenteilchen im Bereich von 1-10 nm, vorzugsweise im Bereich von 2-4 nm,
3. durch die Auswahl der Stabilisatorsubstanzen und
**4.** durch die Dicke und den Wassergehalt der Stabilisatorsubstanzschicht.

Die sehr kleinen superparamagnetische Eindomänenteilchen können aus folgenden Substanzen bestehen: Eisenhydroxid, Eisenoxidhydrat,_γ-Fe₂O₃, Fe₃O₄, aus den Eisenmischoxiden der allgemeinen Formel mMO.n Fe₂O₃, worin M die zweiwertigen Metallionen Fe, Co, Ni, Mn, Be, Mg, Ca, Ba, Sr, Cu, Zn, Pt oder Gemische davon bedeuten, aus den Mischoxiden der allgemeinen Formel m Fe₂O₃.nMe₂O₃, worin Me die dreiwertigen Metallionen Al, Cr, Bi, seltene Erdmetalle oder Gemische davon bedeuten oder Eisen, wobei m und n ganze Zahlen von 1 bis 6 sind. So läßt sich durch die Zusammensetzung und durch die Struktur der Eindomänen-teilchen deren magnetische Suszeptibilität in weiten Grenzen variieren und ein Verhältnis der Relaxivitäten R2/R1 kleiner 5 einstellen. Wichtig für eine Anwendung in der Medizin ist jedoch die Beachtung der Toxizität der einzelnen Bestandteile der Eindomänenteilchen.

Die magnetische Suszeptibilität der erfindungsgemäßen sehr kleinen superparamagnetische Eindomänenteilchen kann auch durch die Zusammensetzung der Eisensalzlösung und die Art der Herstellung in weiten Grenzen, von ca.1 bis 100 EMU/g, eingestellt werden.

überraschend wurde gefunden, daß eine Veränderung der Zusammensetzung der Eisensalzlösung Einfluß auf das Verhältnis der Relaxivitäten R2/R1 hat. Durch eine Vergrößerung des Fe3+/Fe2+-Konzentrations-verhältnises der Eisensalzlösung von 2 auf 10 läßt sich die magnetische Suszeptibilität von ca. 100 auf ca. 20 EMU/g verringern.

Gegebenenfalls kann nach der Fällung der sehr kleinen Eindomänenteilchen durch eine Oxidations- oder Reduktionsreaktion eine Veränderung der Relaxivitäten R2/R1 erreicht werden. Als Redoxmittel sind z.B. Nitrationen, Wasserstoffperoxid, Hydroxylamin, Ascorbinsäure anwendbar.

überraschend wurde gefunden, daß sich die Teilchengröße der superparamagnetischen Eindomänenteilchen noch weiter verringern läßt, wenn die Fällung der Teilchen unter erhöhter Temperatur und gegebenenfalls erhöhtem Druck, mit Basen aus einer wäßrigen oder mit Wasser mischbaren organische Lösungsmittel enthaltenden Eisensalzlösung erfolgt, wobei unmittelbar vor, während oder unmittelbar nach der Fällung eine Lösung aus Stabilisatorsubstanz in Wasser oder in mit Wasser mischbares organische Lösungsmittel zugesetzt wird, oder die Fällung mit einer Mischung aus Stabilisatorsubstanz und Base, oder wenn die Stabilisator-substanz gleichzeitig Base ist, nur durch Zugabe der organischen Base, erfolgt. Eine Fällungstemperatur von 50 bis 120°C kann als vorteilhaft angegeben werden. Bei Temperaturen über 100°C wird im Autoklaven gearbeitet. Die Zugabe der Stabilisatorsubstanz unmittelbar vor, während oder unmittelbar nach der Fällung verhindert die oben angeführten Redoxreaktionen und liefert sehr kleine superparamagnetische Eindomänenteilchen.

Die Teilchengröße der sehr kleinen superparamagnetischen Eindomänenteilchen liegen im Bereich von 1 bis 10 nm, insbesondere im Bereich des mittleren Teilchendurchmessers d50 von 2 bis 4 nm. Die angegebenen Teilchengrößen von 1 bis 10 nm, mit einem mittleren Teilchendurchmesser d50 von 2-4 nm beziehen sich immer auf die Teilchen ohne Stabilisatorsubstanz.

Mit Verkleinerung der Teilchengröße wird auch die biologische Verträglichkeit besser, die Abbaugeschwindigkeit im Körper erhöht. Die Bioverfügbarkeit der sehr kleinen superparamagnetischen Teilchen im Körper beträgt, je nach Teilchengröße und Zusammensetzung der Stabilisatorsubstanzen, einige Stunden bis Tage, d.h. das retikuloendotheliale System bindet die sehr kleinen superparamagnetischen Teilchen relativ langsam.

Der Erfindung liegt weiterhin die Aufgabe zugrunde, den Bereich der Substanzen, die die sehr kleinen superparamagnetischen Eindomänenteilchen gegen Aggregation und Sedimentation stabilisieren sollen, zu erweitern, um die physikochemischen und physiologischen Eigenschaften der entstehenden Magnetteilchen den jeweiligen Anwendungsgebieten optimal anpassen zu können, wobei diese Substanzen stabil und leicht herstellbar sein sollen.

Überraschend wurde gefunden, daß sich für die Stabilisierung der sehr kleinen superparamagnetischen Eindomänenteilchen auch kleine Moleküle, und nicht nur wie die nach dem Stand der Technik bekannten relativ großen Polymer- oder Makromoleküle, eignen.

So wurde gefunden, daß sich aliphatische Di- und Polycarbonsäuren und deren Substitutionsprodukte und Derivate, wie Äpfelsäure, Weinsäure, Zitronensäure, Asparaginsäure, als Stabilisatorsubstanzen für die sehr kleinen superparamagnetischen Eindomänenteilchen eignen.

Die erfindungsgemäßen sehr kleinen superparamagnetischen Eindomänenteilchen, die z.B. mit Zitronensäure stabilisiert sind, haben wesentlich kleinere Durchmesser als die bisher hergestellten kleinsten superparamagnetischen Eisenoxide, die mit einer Polymerbeschichtung versehen sind. So lassen sich stabilisierte superparamagnetische Eindomänenteilchen mit einem Bereich des mittleren Teilchendurchmessers d50 von 2 bis 4 nm herstellen, die durch ein 100.000 D-Filter filtrierbar sind, also einen mittleren Teilchendurchmessers der stabilisierten Teilchen von 4 bis 8 nm aufweisen, Das Verhältnis der Relativitäten R2/R1 kann so auf Werte zwischen 1 und 3 verringert werden.

Damit verlängert sich die Blut-Halbwertszeit der neuen sehr kleinen superparamagnetischen Eindomänen-teilchen gegenüber den bisherigen Teilchen wesentlich und die möglichen Einsatzbereiche, z.B. für die T1-gewichtete NMR-Tomographie in der Angiographie, Lymphographie, Thromben- und Tumor-Diagnostik, erweitern sich.

Überraschend wurde auch gefunden, daß die Art und Menge der Stabilisatorsubstanzen das Verhältnis der Relaxivitäten R2/R1 der superparamagnetische Eindomänenteilchen beeinflussen. So läßt sich R2/R1 verringern, wenn die Hydrophilie und damit der Hydratisierungsgrad der Stabilisatorschicht ansteigt. Die Menge der Stabilisatorsubstanz ist mitbestimmend für die Teilchengröße. Mit steigender Konzentration an Stabilisatorsubstanz wird der Teilchendurchmesser kleiner.

Die physikochemischen und physiologischen Eigenschaften der entstehenden sehr kleinen superpara-magnetischen Eindomänenteilchen lassen sich den jeweiligen Anwendungsgebieten optimal anpassen, wenn auf deren Oberfläche gegebenenfalls zusätzliche Stabilisatorsubstanzen gebunden werden. Diese zusätzliche Stabilisatorsubstanzen können ausgewählt werden unter mono- und polyhydroxylgruppenhaltigen aromatischen Substanzen, wie z.B. Benzenoiden, Cumarinen, Lignanen, Terphenylen, Flavonoiden, Tanninen, Xanthonen, Benzophenonen, Naphthalenen, Naphthochinonen, Anthrachinonen, Anthracyclinen, polycyclischen kondensierten aromatischen Verbindungen und deren Derivaten; aminosäurenhaltigen Substanzen, wie z.B. Albuminen, Globulinen Oligopeptiden, Polypeptiden, Denaturierungsprodukten von Proteinen und Proteiden, wie Gelatine, Kasein-Hydrolysate, Gluteline; thiogruppenhaltigen Substanzen, wie z.B. Mercaptopurin, -cytosin, -guanin, -uracil, -thymin, -hypoxanthin, sowie deren Mercapto-nucleoside und Mercapto-desoxynucleoside; silikatgruppenhaltigen Substanzen der Orthokieselsäure und deren Kondensationsprodukte mit zwei und mehrwertigen anorganischen Ionen und organischen Säuren, wie z.B. Phytinsäure, Alginsäure, Gallussäure; phosphatgruppenhaltigen Substanzen der Ortho- oder Metaphosphorsäure und deren Kondensationsprodukte, wie z.B. Pyrophosphorsäure, Polyphosphorsäuren, Cyclophosphate und deren Heterokondensationsprodukte, und deren Reaktionsprodukte mit basische Gruppen enthaltenden organischen Verbindungen, wie Spermin, Spermidin, Polyethylenimin, Protamine, Oxygelatine und deren Derivate; organischen Substanzen der Gruppe der phosphat-, diphosphat-, polyphosphat-, thiophosphat-, phosphonat-, thiophosphonat-, carboxylat-, sulfat-, sulfonat-, mercapto-, carboxylat-, silantriolgruppenhaltigen Kohlenhydrate, Polyalkylenglykole, Alkyl-, Aryl-, und/oder Alkyl-aryl-polyethylenglykole; phosphatgruppenhaltigen Nucleotiden, deren Oligomere oder deren Polymere; stickstoffhaltigen Polysacchariden, wie Mucopolysacchariden, Glykoproteiden, Chitinen und deren Derivaten.

Die Stabilisatorsubstanzen sind nach dem Stand der Technik herstellbar oder können käuflich erworben werden.

An die Stabilisatormoleküle können alle gewebespezifischen Bindungssubstanzen, wie z.B. Antigene, Antikörper, Ribonucleinsäuren, Desoxyribonucleinsäuren, Ribonucleinsäuresequenzen, Desoxyribonucleinsäuresequenzen, Haptene, Avidin, Streptavidin, Protein A, Protein G, Endotoxin-bindende Proteine, Lectine, Selectine, Oberflächenproteine von Organellen, Viren, Mikroben, Algen, Pilze; alle pharmakologisch wirksame Substanzen, wie z.B. Antitumorproteine, Enzyme, Antitumorenzyme, Antibiotika, Pflanzenalkaloide, Alkylierungsreagenzien, Antimetaboliten, Hormone und Hormonantagonisten, Interleukine, Interferone, Wachstumsfaktoren, Tumornekrosefaktoren, Endotoxine, Lymphotoxine, Urokinase, Streptokinase, Plasminogen-streptokinase-Aktivator-Komplex, Gewebe-Plasminogen-Aktivatoren, Desmodus-Plasminogen-Aktivatoren, Makrophagen-Akti- vierungs-Körper, Antisera, Blut- und Zellbestandteile und deren Abbauprodukte und Derivate, Zellwandbestandteile von Organellen, Viren, Mikroben, Algen, Pilze und deren Abbauprodukte und Derivate, Proteaseninhibitoren, Alkylphosphocholine, radioaktive Isotope enthaltende Substanzen, Tenside, kardiovaskulare Pharmazeutika, Chemotherapeutika, gastrointestinale Pharmazeutika, Neuropharmazeutika;
alle pharmakologisch wirksamen Zellen, wie z.B. Organellen, Viren, Mikroben, Algen, Pilze, insbesondere Erythrozyten, Thrombozyten, Granulozyten, Monozyten, Lymphozyten, Langerhans'sche Inseln;
alle pharmakologisch wirksame Komplexbildner, wie z.B. Polycarbonsäuren, Polyaminosäuren, Porphyrine, Katecholamine;
alle zellfusionvermittelnden Substanzen, wie z.B. Polyethylenglykole, Alkyl-, Aryl- und Alkyl-aryl-polyethylenglykole und deren Derivate;
alle gentransfervermittelnden Substanzen, wie z.B. von Polyethylenglykol und deren Derivaten; Polyaminverbindungen, wie z.B. Polyethylenimin, Spermin, Spermidin, Protaminsulfat; sowie Gemische davon gebunden werden.

Nach dem Stand der Technik gibt es zwei Möglichkeiten superparamagnetische Teilchen, die durch Fällungsreaktionen hergestellt werden, gegen Aggregation und Sedimentation zu stabilisieren.
1. die superparamagnetische Teilchen werden durch Fällung aus den Eisensalzlösungen hergestellt, gereinigt und anschließend mit den entsprechenden Stabilisatorsubstanz stabilisiert oder
2. die Stabilisatorsubstanzen werden mit den Eisensalzlösungen gemischt und die Mischung auf die entsprechende Fällungstemperatur erwärmt und die Fällung durchgeführt.

Da das Gemisch der Fe3+/Fe2+-Salzlösung eine reaktives Redoxsystem darstellt, können Teile der Stabilisatorsubstanz oxidiert oder reduziert werden und das Fe3+/Fe2+-Konzentrationsverhältnis verändert sich unreproduzierbar. Viele mögliche Stabilisatorsubstanzen oder auch viele organische Anionen der Eisensalzlösungen bilden mit den Eisensalzen Komplexe, die ebenfalls zu nichtmagnetischen oder unreproduzierbaren Fällungsprodukten führen.

Das Verfahren zur Herstellung von sehr kleinen superparamagnetischen Eindomänenteilchen mit vergrößerter R1-Relaxivität und mit Oberflächen-Stabilisatorsubstanzen, wobei die Fällung der Teilchen mit Basen, wie z.B.mit Alkalilauge, Ammoniakwasser oder organischen Basen, aus einer Eisensalzlösung bei erhöhter Temperatur im Bereich von 50 bis 120°C erfolgt. Bei Temperaturen über 100°C wird im Autoklaven gearbeitet. Die Fällung kann auch mit einer Mischung aus Stabilisatorsubstanz und Base oder, wenn die Stabilisatorsubstanz gleichzeitig Base ist, nur durch Zugebe der organischen Base vorgenommen werden. Organische Basen, die gleichzeitig als Stabilisatorsubstanz fungieren, sind z.B. Polyethylenimin, Spermin, Spermidin oder Protamin. Der Zusatz einer gelösten Stabilisatorsubstanz, die aus aliphatische Di- und Polycarbonsäuren und deren Substitutionsprodukte und Derivate, wie z.B. Äpfelsäure, Weinsäure, Zitronen-säure, Asparaginsäure, bestehen, zur Eisensalzlösung erfolgt während der Fällung oder unmittelbar nach der Fällung in einer Menge von 5 bis 100 Gew-%, bezogen auf die Eindomänenteilchenmenge.

In dem gefällten Produkt kann durch Zugabe eines Oxidations- oder Reduktionsmittels das Konzentrationsverhältnis Fe3+/Fe2+ im Bereich von eins bis unendlich (γ-Fe₂O₃) eingestellt werden; um die gewünschte Veränderung der Relaxivitäten R2/R1 einzustellen. Die so hergestellte Dispersion von stabilisierten sehr kleinen superparamagnetischen Teilchen wird abgekühlt und z.B. mit Salzsäure neutralisiert. Die Dispersion wird so lange dialysiert, bis die elektrische Leitfähigkeit des Filtrates < 10 mS/cm beträgt.

Bei einigen Stabilisatorsubstanzen benötigt man einen stärkeren Energieeintrag, wie z.B.eine Einwirkung von Ultraschall, um stabile Dispersionen zu erhalten. Die stabilen Dispersionen können noch größere oder schwach aggregierten superparamagnetischen Eindomänenteilchen enthalten, die sich leicht von den sehr kleinen superparamagnetischen Teilchen durch deren Sedimentation in einem Magnetfeld oder durch Zentrifugation abtrennen lassen. Gegebenenfalls können weitere Substanzen der Gruppe der zusätzlichen Stabilisatorsubstanzen, gewebespezifischen Bindungssubstanzen, pharmakologisch wirksamen Substanzen, pharmakologisch wirksamen Zellen, pharmakologisch wirksamen Komplexbildnern, zellfusionvermittelnden Substanzen und gentransfervermittelnden Substanzen; sowie Gemischen davon, an die stabilisierten sehr kleinen superparamagnetischen Eindomänenteilchen angekoppelt werden.

An Beispielen sollen die Herstellung der erfindungsgemäßen sehr kleinen superparamagnetischen Teilchen erläutert werden:

### Beispiel 1:

Eisen(III)-chlorid (270 g) und Eisen(II)-chlorid(119 g) werden in 1 1 dest. Wasser unter Rühren gelöst und unter Sauerstoffausschluß auf 100°C erwärmt. Durch Zugabe einer Mischung von Ammoniakwasser und 35g Zitronensäure wird unter Rühren der pH-Wert der Lösung auf 10 eingestellt und 10 min gekocht. Danach wird die Dispersion auf ca.20°C abgekühlt, mit Salzsäure auf den pH 7,0 eingestellt und mit dest. Wasser dialysiert, bis das Dialysat eine elektrische Leitfähigkeit von < 10 mS/cm besitzt, und 20 min mit Ultraschall von 300 W Leistung dispergiert. Zur Entfernung größerer oder schwach aggregierter superparamagnetischer Teilchen wird die Dispersion 10 min bei 10.000 U/min zentrifugiert. Die sehr kleinen superparamagnetischen Teilchen können mit physiologischer Kochsalzlösung gemischt als positives i.v. Kontrastmittel für die NMR-Diagnostik eingesetzt werden.

### Beispiel 2:

100 ml der Dispersion der sehr kleinen superparamagnetischen Eindomänenteilchen von Beispiel 1, mit einer magnetischen Sättigungsinduktion von 10 mT, werden mit einer Lösung, bestehend aus 4 g Methoxy--polyethylenglykol-phosphat (MG 1000), 1 g Rutin und 50 ml Methanol gemischt und das Methanol im Vakuum abdestilliert. Die Dispersion wird mit Wasser auf 100 ml aufgefüllt, mit Natronlauge auf den pH-Wert von 7,0 eingestellt, 10 min mit Ultraschall von 100 W Leistung dispergiert und zur Entfernung größerer oder schwach aggregierter superparamagnetischer Eindomänenteilchen 10 min bei 10.000 U/min zentrifugiert. Die sehr kleinen superparamagnetischen Teilchen können als positives i.v. Kontrastmittel für die Angiographie in der NMR-Diagnostik eingesetzt werden.

### Beispiel 3;

20 ml der Dispersion der sehr kleinen superparamagnetischen Eindomänenteilchen von Beispiel 2, mit einer magnetischen Sättigungsinduktion von 1 mT, werden mit einer Lösung von 10 mg Doxorubicin in 10 ml physiologische Kochsalzlösung gemischt. Diese sehr kleinen superparamagnetischen Teilchen sind für die Anreicherung in Tumoren und deren Schädigung geeignet.

### Beispiel 4:

Eisen(III)-chlorid (50,3 g) und Eisen(II)-sulfat(139 g)werden in 1 l dest. Wasser unter Rühren gelöst und unter Sauerstoffausschluß auf 85°C erwärmt. Unter Zutropfen von 25%-iger Ammoniumhydroxydlösung wird ein pH-Wert von 10,5 eingestellt. Sofort nach der Fällung wird die Dispersion mit einer Lösung aus 5g L-Asparaginsäure und 25 g Weinsäure in 500 ml Wasser versetzt und 20 min bei 85°C gerührt. Danach wird die Dispersion auf ca. 20°C abgekühlt, mit Salzsäure auf den pH-Wert von 7,0 eingestellt, mit 20 ml 30%-igem Wasserstoffperoxid versetzt und so lange gerührt, bis keine Gasentwicklung mehr auftritt. Die Dispersion wird 20 min mit Ultraschall von 300 W Leistung dispergiert und anschließend dialysiert, bis das Dialysat eine elektrische Leitfähigkeit von < 10 mS/cm besitzt. Zur Entfernung größerer oder schwach aggregierter superparamagnetischer Teilchen wird die Dispersion 10 min bei 10.000 U/min zentrifugiert. Die sehr kleinen superparamagnetischen Teilchen können als positives i.v. Kontrastmittel für die Angiographie in der NMR-Diagnostik eingesetzt werden.

### Beispiel 5:

100 ml der Dispersion der sehr kleinen superparamagnetischen Eindomänenteilchen von Beispiel 4, mit einer magnetischen Sättigungsinduktion von 5 mT, werden mit einer Lösung von 4 g Methoxy-polyethylenglykol-phosphat (MG 2000) und 0,4g Lauryloxy--polyethylenglykol-phosphat (MG 1000) in 50 ml Wasser gemischt. Die sehr kleinen superparamagnetischen Teilchen können mit physiologischer Kochsalzlösung gemischt als Kontrastmittel für die Lymphographie und für die Tumordiagnostik in der NMR-Diagnostik eingesetzt werden.

### Beispiel 6:

100 ml der Dispersion der sehr kleinen superparamagnetischen Eindomänenteilchen von Beispiel 4, mit einer magnetischen Sättigungsinduktion von 5 mT, werden mit einer Lösung von 1 g Oxygelatine in 50 ml Wasser gemischt. Die sehr kleinen superpara-magnetischen Teilchen können mit physiologischer Oxygelatinelösung gemischt als Kontrastmittel für die Lymphographie und für die Tumordiagnostik in der NMR-Diagnostik eingesetzt werden.

### Beispiel 7:

Eisen(III)-chlorid (50,3 g) und Eisen(II)-sulfat(139 g)werden in 1 l dest. Wasser unter Rühren gelöst und unter Sauerstoffausschluß auf 100°C erwärmt. Unter Zutropfen von 25%-iger Ammoniumhydroxydlösung wird ein pH-Wert von 10,5 eingestellt. Sofort nach der Fällung wird die Dispersion mit einer Lösung aus 40 g Äpfelsäure in 500 ml Wasser versetzt und 10 min bei 100°C gerührt. Danach wird die Dispersion auf ca.20°C abgekühlt, mit Salzsäure auf den pH-Wert von 7,0 eingestellt, mit 20 ml 30%-igem Wasserstoffperoxid versetzt und so lange gerührt, bis keine Gasentwicklung mehr auftritt. Die Dispersion wird 20 min mit Ultraschall von 300 W Leistung dispergiert und anschließend dialysiert, bis das Dialysat eine elektrische Leitfähigkeit von < 10 mS/cm besitzt. Zur Entfernung größerer oder schwach aggregierter superparamagnetischer Teilchen wird die Dispersion 10 min bei 10.000 U/min zentrifugiert. Die sehr kleinen superparamagnetischen Teilchen können als positives i.v. Kontrastmittel für die Angiographie in der NMR-Diagnostik eingesetzt werden.

### Beispiel 8:

100 ml der Dispersion der sehr kleinen superparamagnetischen Eindomänenteilchen von Beispiel 7, mit einer magnetischen Sättigungsinduktion von 5 mT, werden mit einer Lösung von 4g Spermidin in 50 ml Wasser gemischt. Die sehr kleinen superparamagnetischen Teilchen können mit Lösungen von phosphatgruppenhaltige Nucleotide, deren Oligomere oder deren Polymere gemischt zum Gentransfer eingesetzt werden.

Typische Analysendaten der sehr kleinen superparamagnetischen Eindomänenteilchen sind:

| | |
|---|---|
| Teilchendurchmesser d50 | 4 nm |
| Gesamtdurchmesser mit Stabilisator | 8 nm |
| Eisen(II)-Gehalt | 6 % |
| T1-Relaxivität | 30 l/mmol s |
| T2-Relaxivität | 56 l/mmol s |
| Verhältnis der Relaxivitäten R2/R1 | 1,87 |

Die Hauptanwendungsgebiete der erfindungsgemäßen sehr kleinen superparamagnetischen Eindomänenteilchen liegen auf den Gebieten der NMR-Kontrastmittel für die Angiographie, Lymphographie, Thromben- und Tumordiagnostik, der Tumorschädigung, der Thrombenauflösung, der Immunsteigerung, der Zellfusionvermittlung oder des Gentransfers, wobei auch hier die Wirksamkeit der Tumorschädigung, der Thrombenauflösung, der Zellfusion und des Gentransfers mit der NMR-Diagnostik untersucht werden kann.

Die sehr kleinen superparamagnetischen Eindomänenteilchen sind für eine Tumordiagnostik einsetzbar, da bei ihrer Injektion in den Blutkreislauf eine Anreicherung, besonders der mit Methoxy-polyethylenglykol--phosphat oder -phosphonat stabilisierten sehr kleinen superparamagnetischen Eindomänenteilchen, in den Tumoren zu beobachten ist.

Bei Kopplung von pharmakologisch wirksamen Substanzen an die sehr kleinen superparamagnetischen Eindomänenteilchen kann deren Konzentration am Wirkungsort erhöht werden, insbesondere bei den mit Methoxy-polyethylenglykol-phosphat oder -phosphonat stabilisierten sehr kleinen superparamagnetischen Eindomänenteilchen oder beim Einsatz tumorspezifischer Antikörper. Dieser Umstand hat für die Krebstherapie Bedeutung, da die zur Chemotherapie von Tumoren eingesetzten Substanzen sehr starke Nebenwirkungen auf den gesamten Organismus ausüben und bei einer Anreicherung am Wirkungsort der übrige Körper weniger stark mit Zytostatika belastet wird.

In Tierversuchen konnten gute Effekte als parenterales Positivkontrastmittel bei T1-gewichteter NMR-Tomographie, wie z.B. für den Blutkreislauf, für die Thromben- und Tumordiagnostik, für die Abbildung des Magen-Darm-Traktes, sowie als antikörperspezifische Kontrastmittel, gefunden werden. Hier wirkt sich die große Bluthalbwertzeit positiv aus, da das retikuloendotheliale System die Teilchen nur langsam aufnimmt und die Teilchen, besonders wenn sie mit Antikörpern gekoppelt sind, über einen längeren Zeitraum im Blutkreislauf beweglich sind und sich so verstärkt am Bindungsort aufkonzentrieren lassen.
Bei T2-gewichteter NMR-Tomographie liefern die sehr kleinen superparamagnetischen Eindomänenteilchen noch einen guten Negativkontrast für Leber, Milz, Knochenmark und Lymphknoten.
Die Mengen an sehr kleinen superparamagnetischen Eindomänenteilchen liegen bei der Anwendung als parenterales Kontrastmittel für die NMR bei ca. 5 bis 20 mM Fe/kg Körpergewicht und bei der Anwendung als orales Kontrastmittel für die MRI bei ca.10 mM Fe/kg Körpergewicht.

## Patentansprüche

1. superparamagnetische Eindomänenteilchen mit vergrößerter R1-Relaxivität und mit Oberflächen-Stabilisatorsubstanzen, **dadurch gekennzeichnet, daß** Teilchen aus Eisenhydroxid, Eisenoxidhydrat, Eisenoxid, Eisenmischoxid oder Eisen eine Teilchengröße haben im Bereich zwischen 1 und 10 Nanometer, mit einem mittleren Teilchendurchmesser d₅₀ von 2 bis 4 Nanometer; eine vergrößerte R₁-Relaxivität im Bereich von 2 bis 50 haben; ein Verhältnis der Relaxivitäten R₂/R₁ kleiner 5 haben; und auf ihrer Oberfläche Stabilisatorsubstanzen tragen, ausgewählt unter aliphatischen Di- und Polycarbonsäuren, deren Substitutionsprodukten und Derivaten; die eine Aggregation und Sedimentation im Schwerefeld oder in einem Magnetfeld verhindern; und gegebenenfalls einen Gehalt an zusätzlicher Stabilisatorsubstanz, gewebespezifischer Bindungssubstanz, pharmakologisch wirksamer Substanz, pharmakologisch wirksamen Zellen, pharmakologisch wirksamen Komplexbildner, zellfusionvermittelnder Substanz oder gentransfervermittelnder Substanz; sowie Gemischen davon aufweisen.

2. Superparamagnetische Teilchen nach Anspruch 1, **dadurch gekennzeichnet, daß** die Teilchengröße der sehr kleinen superpara-magnetischen Eindomänenteilchen im Bereich von 1 bis 5 nm liegt, und daß die sehr kleinen superparamagnetischen Eindomänenteilchen aus Eisenhydroxid; Eisenoxidhydrat; γ-Fe₂O₃; Fe₃O₄; aus den Eisenmischoxiden der allgemeinen Formel mMO·nFe₂O₃, worin M die zweiwertigen Metallionen Fe, Co, Ni, Mn, Be, Mg, Ca, Ba, Sr, Cu, Zn, Pt oder Gemische davon bedeuten; aus den Mischoxiden der allgemeinen Formel m Fe₂O₃·nMe₂O₃, worin Me die dreiwertigen Metallionen Al, Cr, Bi, seltene Erdmetalle oder Gemische davon bedeuten; oder Eisen bestehen, wobei m und n ganze Zahlen von 1 bis 6 sind.

3. Superparamagnetische Teilchen nach Anspruch 1 oder 2, **dadurch gekennzeichnet, daß** die sehr kleinen superparamagnetischen Eindomänenteilchen auf ihrer Oberfläche Stabilisatorsubstanzen tragen, die aus der Gruppe ausgewählt sind, die aus aliphatischen Di- und Polycarbonsäuren und deren Substitutionsprodukten und Derivaten, wie Äpfelsäure, Weinsäure, Zitronensäure, Asparaginsäure, sowie Gemischen davon bestehen.

4. Superparamagnetische Teilchen nach einem der Ansprüche 1 bis 3, **dadurch gekennzeichnet, daß** die sehr kleinen superparamagnetischen Eindomänenteilchen auf ihrer Oberfläche zusätzlich zu den Stabilisator-substanzen, die aus der Gruppe der aliphatischen Di- und Polycarbonsäuren und deren Substitutionsprodukten und Derivaten ausgewählt sind, noch Stabilisatorsubstanzen gebunden tragen, die ausgewählt sind unter mono- und/oder polyhydroxylgruppenhaltige Substanzen, wie glycerin- und polyglyceringruppenhaltige Substanzen, deren Substitutionsprodukte und Derivate; mono- und/oder polyhydroxylgruppenhaltige aromatischen Substanzen, wie aromatische Benzenoide, Cumarine, Lignane, Terphenyle, Flavonoide, Tannine, Xanthone, Benzophenone, Naphthalene, Naphthochinone, Anthrachinone, Anthracycline, polycyclische kondensierte aromatische Verbindungen und deren phosphat-, diphosphat-, polyphosphat-, thiophosphat-, phosphonat-, thiophosphonat-, carboxylat-, sulfat-, mercapto- oder silantriolgruppenhaltigen Derivate; aminosäurenhaltige Makromoleküle, wie Oligopeptide, Polypeptide, Proteine, Proteide sowie deren Derivate und Denaturierungsprodukte; thiogruppenhaltige Substanzen, ausgewählt unter Biotin, Mercaptopurin, -cytosin, -guanin, -uracil, -thymin, -hypoxanthin, sowie deren Mercapto-nucleoside und Mercapto-desoxynucleoside; silikatgruppenhaltige Substanzen der Orthokieselsäure und deren Kondensationsprodukte mit zwei und mehrwertigen anorganischen Ionen, organischen Säuren und Basen: phosphatgruppenhaltige Substanzen der Ortho- oder Metaphosphorsäure und deren Kondensations- sowie Heterokondensationsprodukte und wasserunlösliche Salzverbindungen mit anorganischen Ionen und basische Gruppen enthaltenden organischen Verbindungen, wie Spermin, Spermidin, Polyethylenimin, Protamine, Oxygelatine und deren Derivate; Substanzen der Gruppe der phosphat-, diphosphat-, polyphosphat-, thiophosphat-, phosphonat-, thiophosphonat-, carboxylat-, sulfat-, sulfonat-, mercapto-, silantriol-, trialkoxysilangruppenhaltigen Kohlenhydrate, Polyethylenglykole, Polyalkylenglykole, Alkyl-, Aryl-, Alkyl-aryl-polyethylenglykole, Block-Copolymerisate aus Polyethylenglykol (PEG) und Polypropylenglykol (PPG), wie den Block-Copolymerisaten (PEG)n-(PPG)m, (PEG)n-(PPG)m-(PEG)n, (PPG)m-(PEG)n-(PPG)m; stickstoffhaltige Polysaccharide, wie Mucopolysaccharide, Glykoproteide, Chitine, sowie deren Derivate und Denaturierungsprodukte; phosphatgruppenhaltige Nucleotide, deren Oligomere oder deren Polymere; sowie Gemischen davon gebunden tragen.

5. Verfahren zur Herstellung von superparamagnetischen Eindomänenteilchen mit ausgeprägter veränderter R₁-Relaxivität und mit Oberflächen-Stabilisatorsubstanzen, wobei die Fällung der Teilchen mit anorganischen und organischen Basen aus einer Eisensalzlösung erfolgt, **dadurch gekennzeichnet, daß** die Fällung bei erhöhter Temperatur im Bereich von 50 bis 120°C erfolgt und der Zusatz einer gelösten Stabilisatorsubstanz, die aus aliphatischen Di- und Polycarbonsäuren und deren Substitutionsprodukten und Derivaten, wie Äpfelsäure, Weinsäure, Zitronensäure, Asparaginsäure, sowie Gemischen davon bestehen, zur Eisensalzlösung während der Fällung oder unmittelbar nach der Fällung in einer Menge von 5 bis 100 Gew-%, bezogen auf die Eindomänenteilchenmenge, erfolgt; und in dem gefällten Produkt ein Konzentrationsverhältnis Fe³⁺/Fe²⁺ im Bereich von eins bis unendlich, durch Zugabe eines Reduktions- oder Oxidationsmittels, eingestellt wird; danach das Fällungsprodukt gereinigt und die gewünschte Teilchengröße abgetrennt wird; und gegebenenfalls weitere Substanzen der Gruppe der Stabilisatorsubstanzen, gewebespezifischen Bindungssubstanzen, pharmakologisch wirksamen Substanzen, pharmakologisch wirksamen Zellen, pharmakologisch wirksamen Komplexbildnern, zellfusionvermittelnden Substanzen und gentransfervermittelnden Substanzen; sowie Gemischen davon angekoppelt werden.

6. Pharmakologische Zubereitung, bestehend aus einem pharmakologisch annehmbaren Träger und superparamagnetischen Teilchen nach Anspruch 1 mit einer Teilchengröße im Bereich zwischen 1 und 10 Nanometer, gegebenenfalls in Verbindung mit einer gewebespezifischen Bindungssubstanz, einer pharmakologisch wirksamen Substanz, einer pharmakologisch wirksamen Zelle, einem pharmakologisch wirksamen Komplexbildner, einer zellfusionvermittelnden Substanz und/oder einer gentransfervermittelnden Substanz; sowie Gemischen davon.

7. Verwendung von superparamagnetischen Eindomänenteilchen mit vergrößerter R1-Relaxivität und mit Oberflächen-Stabilisatorsubstanzen zur Herstellung einer pharmakologischen Zubereitung nach Anspruch 6 zur Tumorschädigung, zur Immunsteigerung, zum magnetischen drug targeting, zur Zellfusion, zum Gentransfer, als Kontrastmittel in der Kernspin- Diagnostik, gegebenenfalls unter Einwirkung von Magnetfeldern.

## Claims

1. Super-paramagnetic single-domain particles having an increased R₁ relaxivity and surface stabilizer substances, comprising particles of iron hydroxide, iron oxide hydrate, iron oxide, mixed iron oxides or iron having a particle size in the range between 1 and 10 nanometers, with an average particle diameter d₅₀ of two to four nanometers; having an increased R₁ relaxivity in the range of two to fifty; having a R₂/R₁ relaxivity ratio of less than five; and having at their surface stabilizer substances selected from aliphatic di- and polycarboxylic acids, their substitution products and derivatives; preventing aggregation and sedimentation in a gravitational field or in a magnetic field; and optionally containing an additional stabilizer substance, tissue-specific binding substance, pharmacologically active ingredient, pharmacologically active cells, pharmacologically active chelating agent, cell fusion mediating substance or gene transfer mediating substance, as well as mixtures thereof.

2. Super-paramagnetic particles according to Claim 1, wherein the particle size of the very small super-paramagnetic single-domain particles is in the range of 1 to 5 nm, and the very small super-paramagnetic single-domain particles consist of iron hydroxide; iron oxide hydrate; γ-Fe₂O₃; Fe₃O₄; mixed iron oxides of the general formula mMO·nFe₂O₃, where M denotes the divalent metal ions Fe, Co, Ni, Mn, Be, Mg, Ca, Ba, Sr, Cu, Zn, Pt or mixtures thereof; mixed oxides of the general formula mFe₂O₃·nMe₂O₃, where Me denotes the trivalent metal ions Al, Cr, Bi, rare earth metals or mixtures thereof; or iron, where m and n are integers from 1 to 6.

3. Super-paramagnetic particles according to Claim 1 or 2, wherein the very small super-paramagnetic single-domain particles have on their surface stabilizer substances selected from the group consisting of aliphatic di- and polycarboxylic acids and their substitution products and derivatives such as malic acid, tartaric acid, citric acid, aspartic acid as well as mixtures thereof.

4. Super-paramagnetic particles according to one of Claims 1 through 3, wherein the very small super-paramagnetic single-domain particles, in addition to having stabilizer substances selected from the group of aliphatic di- and polycarboxylic acids and their substitution products and derivatives at their surface, also have bound on their surface stabilizer substances selected from substances containing mono- and/or polyhydroxyl groups, such as substances containing glycerol and polyglycerol groups, their substitution products and derivatives; aromatic substances containing mono- and/or polyhydroxyl groups, such as aromatic benzenoids, coumarins, lignans, terphenyls, flavonoids, tannins, xanthones, benzophenones, naphthalenes, naphthoquinones, anthraquinones, anthracyclines, polycyclic condensed aromatic compounds and their derivatives containing phosphate groups, diphosphate groups, polyphosphate groups, thiophosphate groups, phosphonate groups, thiophosphonate groups, carboxylate groups, sulfate groups, mercapto groups or silanetriol groups; macromolecules containing amino acids, such as oligopeptides, polypeptides, proteins, proteids and derivatives thereof and denaturing products; substances containing thio groups, selected from biotin, mercaptopurine, mercaptocytosine, mercaptocytosine, mercaptoguanine, mercaptouracil, mercaptothymine, mercaptohypoxanthine as well as their mercaptonucleosides and mercaptodeoxynucleosides; substances of ortho-silicic acid containing silicate groups and their condensation products with divalent and polyvalent inorganic ions, organic acids and bases; substances of ortho- or metaphosphoric acid containing phosphate groups as well as their condensation products and heterocondensation products and water-insoluble salt compounds with inorganic ions and organic compounds having basic groups, such as spermine, spermidine, polyethyleneimine, protamines, oxygelatin and derivatives thereof; substances from the group of carbohydrates having phosphate groups, diphosphate groups, polyphosphate groups, thiophosphate groups, phosphonate groups, thiophosphonate groups, carboxylate groups, sulfate groups, sulfonate groups, mercapto groups, silanetriol groups or trialkoxysilane groups, polyethylene glycols, polyalkylene glycols, alkyl polyethylene glycols, aryl polyethylene glycols, alkylaryl polyethylene glycols, block copolymers of polyethylene glycol (PEG) and polypropylene glycol (PPG), such as the block copolymers (PEG)ₙ-(PPG)ₘ, (PEG)ₙ-(PPG)ₘ-(PEG)ₙ or (PPG)ₘ-(PEG)ₙ-(PPG)ₘ; polysaccharides that contain nitrogen, such as mucopolysaccharides, glycoproteids, chitins, and their derivatives and denaturing products; nucleotides containing phosphate groups or their oligomers or polymers, and mixtures thereof.

5. A method of producing super-paramagnetic single-domain particles with a marked, altered R₁ relaxivity and with surface stabilizer substances, where the particles are precipitated with organic and inorganic bases from an iron salt solution, wherein precipitation is performed at an elevated temperature in the range of 50°C to 120°C, and a dissolved stabilizer substance consisting of aliphatic di- and polycarboxylic acids and their substitution products and derivatives such as malic acid, tartaric acid, citric acid, aspartic acid as well as mixtures thereof, is added to the iron salt solution during or immediately after precipitation in an amount of 5 to 100 wt%, based on the quantity of single-domain particles; and an Fe³⁺/Fe²⁺ concentration ratio in the range from one to infinity is established in the precipitated product by adding a reducing agent or an oxidizing agent; then the precipitation product is purified and the desired particle size is separated; and optionally additional substances from the group of stabilizer substances, tissue-specific binding substances, pharmacologically active substance, pharmacologically active cells, pharmacologically active chelating agents, cell fusion mediating substances and gene transfer mediating substances, as well as mixtures thereof are coupled [to the particles].

6. A pharmacological preparation consisting of a pharmacologically acceptable carrier and super-paramagnetic particles according to Claim 1 with a particle size in the range between 1 and 10 nanometers, optionally in combination with a tissue-specific binding substance, a pharmacologically active substance, a pharmacologically active cell, a pharmacologically active chelating agent, a cell fusion mediating substance and/or a gene transfer mediating substance; as well as mixtures thereof.

7. Use of super-paramagnetic single-domain particles with increased R₁ relaxivity and with surface stabilizer substances for producing of a pharmacological preparation according to Claim 6 for tumor destruction, for boosting immunity, for magnetic drug targeting, for cell fusion, for gene transfer, as a contrast medium in NMR diagnostics, optionally under the influence of magnetic fields.

## Revendications

1. Particules monodomaine superparamagnétiques à relaxivité R1 accrue et possédant des substances de stabilisation de surface, **caractérisées en ce que** des particules d'hydroxyde de fer, d'oxyde de fer hydraté, d'oxyde de fer, d'oxyde de fer mixte ou de fer ont une taille de particules dans la plage de 1 à 10 nanomètres, avec un diamètre de particules moyen d₅₀ de 2 à 4 nanomètres ; ont une relaxivité R1 accrue dans la plage de 2 à 50 ; ont un rapport des relaxivités R2/R1 inférieur à 5 ; et portent sur leur surface des substances stabilisatrices, choisies parmi des acides di- et polycarboxyliques aliphatiques et leurs produits de substitution et dérivés, qui empêchent une agrégation et une sédimentation dans le champ de gravité ou dans un champ magnétique ; et présentent le cas échéant une teneur en substance stabilisatrice supplémentaire, en substance de liaison tissulaire spécifique, en substance pharmacologiquement active, en cellules pharmacologiquement actives, en agents complexants pharmacologiquement actifs, en substance assurant la médiation de la fusion cellulaire ou en substance assurant la médiation du transfert génique ; ainsi que des mélanges de celles-ci.

2. Particules superparamagnétiques selon la revendication 1, **caractérisées en ce que** la taille des particules monodomaine superparamagnétiques de très petite taille se situe dans la plage de 1 à 5 nm et **en ce que** les particules monodomaine superparamagnétiques de très petite taille se composent d'hydroxyde de fer ; d'oxyde de fer hydraté ; de γ-Fe₂O₃ ; de Fe₃O₄ ; des oxydes de fer mixtes de la formule générale mMO·nFe₂O₃, où M désigne les ions métalliques divalents Fe, Co, Ni, Mn, Be, Mg, Ca, Ba, Sr, Cu, Zn, Pt ou des mélanges de ceux-ci ; des oxydes mixtes de la formule générale m Fe₂O₃·nMe₂O₃, où Me désigne les ions métalliques trivalents Al, Cr, Bi, des terres rares ou des mélanges de ceux-ci ; ou de fer ; et m et n sont des nombres entiers valant de 1 à 6.

3. Particules superparamagnétiques selon la revendication 1 ou la revendication 2, **caractérisées en ce que** les particules monodomaine superparamagnétiques de très petite taille portent sur leur surface des substances stabilisatrices choisies dans le groupe comprenant des acides di- et polycarboxyliques aliphatiques et leurs produits de substitution et dérivés tels que l'acide malique, l'acide tartrique, l'acide citrique, l'acide aspartique, ainsi que des mélanges de ceux-ci.

4. Particules superparamagnétiques selon l'une des revendications 1 à 3, **caractérisées en ce que** les particules monodomaine superparamagnétiques de très petite taille portent, liées sur leur surface en plus des substances stabilisatrices choisies dans le groupe comprenant des acides di- et polycarboxyliques aliphatiques et leurs produits de substitution et dérivés, également des substances stabilisatrices choisies parmi des substances contenant des groupes mono- et/ou polyhydroxyle, par exemple des substances contenant des groupes glycérine et polyglycérine, leurs produits de substitution et dérivés ; des substances aromatiques contenant des groupes mono- et/ou polyhydroxyle, par exemple des benzénoïdes aromatiques, des coumarines, des lignanes, des terphényles, des flavonoïdes, des tannines, des xanthones, des benzophénones, des naphtalènes, des naphtoquinones, des anthraquinones, des anthracyclines, des composés aromatiques condensés polycycliques et leurs dérivés contenant des groupes phosphate, diphosphate, polyphosphate, thiophosphate, phosphonate, thiophosphonate, carboxylate, sulfate, mercapto ou silanetriol ; des macromolécules contenant des acides aminés telles que des oligopeptides, des polypeptides, des protéines, des protides ainsi que leurs dérivés et produits de dénaturation ; des substances contenant des groupes thio, choisies parmi la biotine, les mercaptopurine, -cytosine, -guanine, -uracile, -thymine, -hypoxanthine, ainsi que leurs mercaptonucléosides et mercaptodésoxynucléosides ; des substances contenant des groupes silicates issues de l'acide orthosilicique et leurs produits de condensation avec des ions inorganiques di- et multivalents, des acides et des bases organiques ; des substances contenant des groupes phosphate issues de l'acide ortho- ou métaphosphorique et leurs produits de condensation et hétérocondensation ; et des sels insolubles dans l'eau avec des ions inorganiques et des composés organiques contenant des groupes basiques tels que la spermine, la spermidine, la polyéthylène imine, des protamines, des oxygélatines et leurs dérivés ; des substances du groupe des hydrates de carbone contenant des groupes phosphate, diphosphate, polyphosphate, thiophosphate, phosphonate, thiophosphonate, carboxylate, sulfate, sulfonate, mercapto, silanetriol, trialcoxysilane, des polyéthylène glycols, des polyalkylène glycols, des alkyl-, aryl- et alkylarylpolyéthylène glycols, des copolymères en masse de polyéthylène glycol (PEG) et de polypropylène glycol (PPG), par exemple les copolymères en masse (PEG)n-(PPG)m, (PEG)n-(PPG)m-(PEG)n, (PPG)m-(PEG)n-(PPG)m; des polysaccharides azotés, tels que des mucopolysaccharides, des glycoprotéides, des chitines, ainsi que leurs dérivés et produits de dénaturation ; des nucléotides contenant des groupes phosphate, des oligomères ou des polymères de ceux-ci ; ainsi que des mélanges de ces substances.

5. Procédé de fabrication de particules monodomaine superparamagnétiques à relaxivité R1 nettement modifiée et possédant des substances de stabilisation de surface, dans lequel la précipitation des particules se fait avec des bases inorganiques et organiques à partir d'une solution de sel de fer, **caractérisé en ce que** la précipitation se fait à une température élevée dans la plage de 50 à 120°C et **en ce que** l'ajout d'une substance stabilisatrice dissoute, composée d'acides di- et polycarboxyliques aliphatiques et de leurs produits de substitution et dérivés tels que l'acide malique, l'acide tartrique, l'acide citrique, l'acide aspartique, ainsi que des mélanges de ceux-ci, à la solution de sel de fer se fait pendant la précipitation ou immédiatement après la précipitation en une quantité de 5 à 100% en poids ramenée à la quantité de particules monodomaine ; et **en ce que**, dans le produit précipité, on ajuste un ratio de concentration Fe³⁺/Fe²⁺ dans la plage de 1 à l'infini par l'ajout d'un agent réducteur ou oxydant ; **en ce que** le produit de précipitation est ensuite purifié et la taille de particules souhaitée est séparée ; et le cas échéant **en ce que** d'autres substances du groupe des substances stabilisatrices, des substances de liaison tissulaire spécifique, des substances pharmacologiquement actives, des cellules pharmacologiquement actives, des agents complexants pharmacologiquement actifs, des substances assurant la médiation de la fusion cellulaire et des substances assurant la médiation du transfert génique, ainsi que des mélanges de ces substances y sont couplées.

6. Préparation pharmacologique comprenant un véhicule pharmaceutiquement acceptable et des particules superparamagnétiques selon la revendication 1 ayant une taille de particules dans la plage de 1 à 10 nanomètres, le cas échéant en liaison avec une substance de liaison tissulaire spécifique, une substance pharmacologiquement active, une cellule pharmacologiquement active, un agent complexant pharmacologiquement actif, une substance assurant la médiation de la fusion cellulaire et/ou une substance assurant la médiation du transfert génique ; ainsi que des mélanges de ces substances.

7. Utilisation de particules monodomaine superparamagnétiques à relaxivité R1 accrue et possédant des substances de stabilisation de surface pour la fabrication d'une préparation pharmacologique selon la revendication 6 pour la destruction de tumeurs, pour le renforcement immunitaire, pour le ciblage médicamenteux magnétique, pour la fusion cellulaire, pour le transfert génique, comme agent de contraste dans le diagnostic par RMN, le cas échéant sous l'action de champs magnétiques.
